# EUROPEAN PATENT SPECIFICATION

(11) **EP 4 504 155 B1**
(45) Date of publication and mention of the grant of the patent: **06.05.2026**
(21) Application number: 23720971.3
(22) Date of filing: 04.04.2023
(51) Int. Cl.: A61K 9/50, A61K 9/16, A61K 31/4045, A61P 25/20

(54) **DELAYED-RELEASE GRANULATES OF MELATONIN, COMBINATIONS COMPRISING MELATONIN, PHARMACEUTICAL COMPOSITIONS AND DIETARY SUPPLEMENTS CONTAINING THEM, AND THEIR USE IN THE TREATMENT OF INSOMNIA AND FOR THE REDUCTION OF THE TIME REQUIRED TO FALL ASLEEP**
MELATONIN-GRANULATE MIT VERZÖGERTER FREISETZUNG, MELATONIN ENTHALTENDE KOMBINATIONEN, DIESE ENTHALTENDE PHARMAZEUTISCHE ZUSAMMENSETUNGEN UND NAHRUNGSERGÄNZUNGSMITTEL SOWIE DEREN VERWENDUNG ZUR BEHANDLUNG VON SCHLAFLOSIGKEIT UND ZUR VERKÜRZUNG DER ZUM EINSCHLAFEN ERFORDERLICHEN ZEIT
GRANULES DE MÉLATONINE À LIBÉRATION RETARDÉE, COMBINAISONS COMPRENANT DE LA MÉLATONINE, COMPOSITIONS PHARMACEUTIQUES ET COMPLÉMENTS ALIMENTAIRES LES CONTENANT, ET LEUR UTILISATION DANS LE TRAITEMENT DE L'INSOMNIE ET POUR LA RÉDUCTION DU TEMPS NÉCESSAIRE À L'ENDORMISSEMENT

(30) Priority: 04.04.2022 IT 202200006590
(43) Date of publication of application: 12.02.2025
(73) Proprietor: S.I.I.T. S.r.l.-Servizio Internazionale Imballaggi Termosaldanti, 20090 Trezzano sul Naviglio MI (IT)
(72) Inventor: COSTA, Andrea Domizio, 20090 Trezzano sul Naviglio (MI) (IT); MADARO, Elena, 20090 Trezzano sul Naviglio (MI) (IT); MERICO, Roberto, 20090 Trezzano sul Naviglio (MI) (IT)
(74) Representative: Marben S.r.l.
(86) International application number: PCT/IB2023/053405
(87) International publication number: WO 2023/194899

(56) References cited:
- WO-A1-2013/152852
- WO-A1-2018/106280
- WO-A2-2012/103411
- CN-A- 107 375 322
- CN-A- 114 259 490
- GB-A- 2 260 080

## Description

It is an object of the present invention a delayed-release melatonin granulate, pharmaceutical compositions and dietary supplements comprising said granulate and their use in the treatment of sleep disorders and for the reduction of the time required to fall asleep, particularly of primary insomnia. It is also an object of the present invention to a combination consisting of melatonin, sodium alginate and calcium lactate and its use in the treatment of sleep disorders, particularly primary insomnia.

### Technical Background

The International Classification of Sleep Disorders defines insomnia as the subjective perception of difficulty falling asleep, duration, consolidation, or quality of sleep that occurs despite adequate opportunity for sleep and results in some form of daytime disturbance (e.g., fatigue, decreased energy, daytime sleepiness, and mood disordes).

Insomnia disorders can be primary or secondary. Primary insomnias may have both intrinsic and extrinsic factors involved in their etiology, but they are not considered secondary to another disorder. Secondary forms occur when insomnia is a symptom of a medical or psychiatric disease, another sleep disorder, or results from substance abuse.

The term primary insomnia (used in both the International Classification of Diseases (ICD-10) and the Diagnostic and Statistical Manual of Mental Disorders, Fourth Edition (DSM-IV)) is a disorder identified as chronic when it persists for at least three months with a frequency of at least three times a week. When the disorder meets the symptom criteria but persists for less than three months, it is considered acute short-term insomnia.

Different manifestations of insomnia can occur at different times during the sleep period.

By way of example:
- sleep onset insomnia (or initial insomnia) involves difficulty initiating sleep before bedtime;
- sleep maintenance insomnia (or medium insomnia) involves frequent or prolonged awakenings during the night;
- late insomnia involves early morning awakening with an inability to return to sleep;
- non restorative sleep, a disorder of poor sleep quality that does not leave the individual refreshed upon awakening despite adequate duration, is a common sleep disorder that usually occurs in association with difficulty initiating or maintaining sleep; this disorder can also occur in association with other sleep disorders (e.g., breathing-related sleep disorder).

Impairment of cognitive performance may include difficulties with attention, concentration, and memory, and may even impair the performance of simple manual skills. Associated mood disorders, are generally described as irritability or mood lability and less commonly as depressive or anxious symptoms. Not all individuals with nighttime sleep disorders are anxious or have functional impairment. For example, sleep continuity is often disrupted in healthy elderly people who nevertheless identify as good sleepers. A diagnosis of insomnia disorder should be reserved for those individuals with significant daytime discomfort or impairment related to their nighttime sleep difficulties.

An insomnia disorder is diagnosed if insomnia presents as an independent condition or is in comorbidity with another mental disorder (e.g., major depressive disorder), a medical condition (e.g., pain) or another sleep disorder (e.g., a breathing-related sleep disorder).

The onset of insomnia symptoms can occur at any time in life, but the first episode is most common in young adulthood. Less frequently, insomnia begins in childhood or adolescence. Insomnia is a more prevalent disorder among females than males, with first onset often associated with the birth of a new baby or menopause. Despite a higher prevalence among older females, polysomnographic studies suggest better preservation of sleep continuity and slow-wave sleep in older females than in older males.

Short-term insomnia affects 30% to 50% of the population.

It is estimated that the prevalence of chronic insomnia disorder in industrialized nations is at least 5% to 10%. Chronic insomnia is associated with numerous adverse effects on function, health, and quality of life. Increased rates of absenteeism from work, and work and motor vehicle accidents have also been widely reported. Persistent insomnia has been identified in numerous studies as a significant risk factor for the development of psychiatric disorders, particularly mood disorders. This condition is also associated with an increased risk of relapse for depression and alcoholism, as well as adverse effects in populations with chronic pain. More recent investigations suggest that chronic insomnia is associated with an increased risk of cardiovascular disease. In particular, insomnia with objectively short sleep times is a significant risk factor for the development of hypertension. Regulation (EU) 432/2012 established the list of health claims authorized under Article 13.1 of Regulation (EC) 1924/2006 for vitamins, minerals and other substances. Substances with authorized claims include melatonin.

The above list includes for each substance the "conditions of use" indicating the required quantitative intake for the claim and any "restrictions on use" and/or additional warnings.

That said, it should be considered that the quantitative intakes of a substance for which a health claim is authorized have a "physiological" value because they are aimed at contributing to the normal performance of the body's functions. With regard to melatonin, Regulation (EU) 432/2012 allows two types of claims:
- helps alleviate the effects of *"jet lag"* by indicating on the label to the consumer that the beneficial effect is achieved by taking, just before bedtime, a minimum of 0.5 mg of the substance on the first day of travel and for some days after arrival at the destination;
- contributes to a reduction in the time required to fall asleep by indicating on the label to the consumer that the beneficial effect is achieved by taking, just before bedtime, 1 mg of the substance.

Thus, today, levels of melatonin useful for physiological effects are defined based on EFSA's scientific assessments, and at the same time, there are products based on the same substance on the European Union market that are authorized as medicaments with daily dosages of 2 mg.

Melatonin is a hormone involved in the management of the circadian rhythm, melatonin levels in mammals being high at night and low during the day. Melatonin is for this reason used in the treatment of sleep disorders, in general, and insomnia in particular, and its administration is particularly indicated to induce sleep in individuals who experience severe difficulty falling asleep. However, conventional melatonin formulations are mostly characterized by rapid release and, consequently, a corresponding rapid metabolism of the active ingredient, due to the short half-life of the melatonin molecule. Therefore, these formulations provide a melatonin release peak at supraphysiological levels 1-2 hours after administration and a subsequent gradual return to normal levels. Such immediate-release formulations therefore fail to reproduce the time course of endogenous melatonin.

Documents WO2018/106280 A1 and WO2012/103411 A2 describe granulates comprising melatonin and excipients, but do not describe granulates comprising, in addition to melatonin, sodium alginate and calcium lactate.

Document CN 114259490 A describes a melatonin preparation comprising an immediate-release portion and an enteric-coated sustained-release portion; where, in parts by weight, the immediate-release portion comprises: 0.04-0.12 parts of melatonin, 20-140 parts of diluent. The enteric-coated sustained-release portion comprises: 0.001-0.01 parts of melatonin, 0.4-20 parts of sustained-release material, 12-90 parts of diluent and 0.6-24 parts of coating agent. The diluent consists of at least one from the following: microcrystalline cellulose, lactose, mannitol, white sugar, dextrin, alditol, oligosaccharides, starch, and pregelatinized starch. The extended-release material is at least one from hypromellose, hypromellose, ethyl cellulose, sodium carboxymethyl cellulose, sodium alginate, and acrylic resin. The coating agent is an enteric film coating agent. The outer layer of the slow-release enteric portion is coated with an enteric film coating agent. A granulate comprising, in addition to melatonin, sodium alginate and calcium lactate is not described.

Therefore, it is understood that there is still an ongoing need to provide new and original melatonin compositions that offer an appropriate duration of action and thus enable a longer-lasting effect during sleep and, consequently, better treatment of sleep disorders.

### Purposes of the invention

It is a first purpose of the invention to provide a granulate that offers a controlled, time-prolonged release of a useful, acceptable and effective amount of melatonin for the treated subject.

It is another purpose of the invention to provide a process for preparing the granulate of the invention.

It is another purpose of the invention to provide a pharmaceutical composition and/or dietary supplement comprising the granulate of the invention and its use in the treatment of sleep disorders and for the reduction of the time required to fall asleep.

The invention is also reported and described with reference to Figures 1-2 (A and B) in which photos are shown of the outcome of the tests performed:
Figure 1 shows the behavior of the 2 granulates just poured into HCl 0.1N.
Figure 2A and 2B shows the behavior of the 2 granulates just poured into HCl 0.1N (top view).

### Description of the invention

The Applicant, after a long and intensive research and development activity, has developed a melatonin-based granulate that is easy to administer and free of side effects, allowing its sustained release over time after it is taken, which is optimal for the treatment of sleep disorders and for the maintenance of the state of falling asleep.

Thus, according to one of its aspects, it is an object of the invention a slow and gradual-release granulate that comprises or, alternatively, consists of melatonin, sodium alginate, and calcium lactate.

The alginate salts found in algae are insoluble. A transformation of them into, for example, sodium alginate, or other soluble salts of alginic acid, is necessary.

The Applicant selected, among all soluble alginate salts, sodium alginate and, after many attempts, found it useful to combine sodium alginate with a soluble salt of a bivalent cation.

Among all possible bivalent cations, the Applicant selected Ca2+, both according to affinity and taking into account safety. Of all the soluble salts of a bivalent cation, the choice was made in favor of calcium lactate because it was the result of careful selection reasoning taking into account the physicochemical properties of calcium lactate and the other components present together in the composition of the granulate (sodium alginate and melatonin) that is the object of the present invention. Among all possible calcium salts, calcium lactate was selected because of a number of characteristics that make calcium lactate the salt of choice for the preparation of the melatonin granulate, which is the object of the present invention: i) good solubility in water; ii) pH of the 5% solution of calcium lactate in water ranging from 6 to 8; iii) Ca²⁺ titer ranging from 12% to 18%, preferably ranging from 13.4% to 14.5%.

All components of the granulate of the invention are known to the technique and commercially available.

Preferably melatonin has a titer of 95% or higher, more preferably 98% or higher, even more preferably 99% or higher, e.g. 99.5%.

According to a preferred embodiment, the granulate of the invention comprises melatonin from 1% to 20% by weight of melatonin with respect to the total weight of the granulate, preferably from 1.5% to 15%, more preferably from 2% to 10%, e.g., 2.5%; 3%; 3.5%; 4%; 4.5% or 5%.

According to a preferred embodiment, the granulate of the invention comprise sodium alginate from 20% to 60%, preferably from 40% to 55%, more preferably from 45% to 50%, e.g., about 46%; 47%; 48%; or 49% of sodium alginate with respect to the total weight of the granulate.

According to a preferred embodiment, the granulate of the invention comprises from 30% to 60%, preferably from 40% to 55%, more preferably from 45% to 50%, e.g., about 46%; 47%; 48%; 49%, of calcium lactate.

Preferably, the granulate of the invention comprises:
- from 1% to 20% by weight of melatonin, with respect to the total weight of the granulate, preferably from 1.5% to 15%, more preferably from 2% to 10%;
- from 20% to 60% by weight of sodium alginate, with respect to the total weight of the granulate, preferably from 40% to 55%, more preferably from 45% to 50%, e.g., about 49%; and
- from 30% to 60% by weight of calcium lactate, with respect to the total weight of the granulate, preferably from 40% to 55%, more preferably from 45% to 55%, e.g., about from 49% to 50%.

According to another of its aspects, it is an object of the invention a process for preparing the granulate of the invention that comprises or, alternatively, consists of the following steps:
a. a wet granulation of a mixture of (i) melatonin, in an amount by weight from 1% to 20%, e.g., about 2%, or 10% by weight, with respect to the total weight of the granulate, (ii) sodium alginate, in an amount by weight from 20% to 60%, e.g., about 45%, or 49% by weight, with respect to the total weight of the granulate, and (iii) calcium lactate, in an amount by weight from 30% to 60%, e.g. 45%, or 49% by weight, with respect to the total weight of the granulate, to form granules intended to be coated to give coated granules;
b. a coating of the granules obtained from step (a) through two sequential steps to give coated granules:
   b' a granulation of the granules from passage (a) with calcium lactate; and
   b" a granulation of the granules from the passage (b') with sodium alginate .

In step (a) a wet granulation is made by mixing from 1% to 20% by weight of melatonin with from 20% to 60% by weight of sodium alginate, and with from 30 to 60% by weight of calcium lactate and water, according to known techniques.

The granules obtained from step (a) are dried, e.g., in an oven, preferably at a temperature from 40°C to 80°C, more preferably from 50°C to 70°C, e.g., at 60°C, and for a time preferably from 2 hours to 12 hours, more preferably from 4 hours 10 hours, e.g., 8 hours. Preferably, the dried granules undergo a calibration step using, for example, a mesh or sieve with a mesh size from 0.5 mm to 2.5 mm, preferably from 1 mm to 2 mm, e.g., 1, 2 mm, or 1.4 mm, or 1.6 mm.

Said dried granules from step (a) are, then, coated by step (b) through the two subsequent granulations (b') and (b"); first the granules are treated with calcium lactate in step (b') to obtain calcium lactate-coated granules, then the latter are further coated with sodium alginate in step (b"). The granules obtained after granulation in each of steps (a), (b') and (b") are dried according to known techniques.

According to a preferred embodiment, the granulations of steps (b) are either fluidized bed or slurry granulations. Alternatively, the process for preparing the granulate of the invention can be accomplished by a single granulation of a mixture of melatonin, sodium alginate and calcium lactate, followed by a single coating step.

According to another of its aspects, it is an object of the invention a process for preparing the granulate of the invention that comprises or alternatively consists of the following steps:
a. a wet granulation of a mixture of (i) melatonin, in an amount by weight from 1% to 20%, e.g., about 2%, or 10% by weight, with respect to the total weight of the granulate, (ii) sodium alginate, in an amount by weight from 20% to 60%, e.g., about 45%, or 49% by weight, with respect to the total weight of the granulate, and (iii) calcium lactate, in an amount by weight from 30% to 60%, e.g. 45%, or 49% by weight, with respect to the total weight of the granulate, to form granules intended to be coated to give coated granules;
b. a coating of the granules obtained from step (a) through one coating step with a mixture of sodium alginate and calcium lactate, to give coated granules.

According to a preferred embodiment, the granules have a size ranging from 0.8 mm to 1.5 mm, preferably from 1 mm to 1.4 mm.

Some details of the process of the present invention are provided in the Experimental Section below, for illustrative purposes only.

The granulate of the invention allows for a prolonged time release of melatonin after it is taken by a subject in need of melatonin. Said granulate can be used as such, e.g., it can be included in gelatin capsules to be administered for the treatment of sleep disorders and for the reduction of the time required to fall asleep.

Preferably, the granulate of the invention can be used for the preparation of pharmaceutical compositions and dietary supplements with controlled-release of melatonin.

According to another of its aspects, it is also an object ot the invention an oral pharmaceutical composition and/or dietary supplement comprising the granulate of the invention together with one or more conventional excipients and/or vehicles.

According to a preferred embodiment, the compositions of the invention are in the form of gummy candies, chewable tablets, capsules or chewing gum.

For the preparation of the preferred compositions of the invention, the granulate of the invention may be mixed with excipients known to the person skilled in the art for making gummy candies, chewable tablets, capsules or chewing gum. By way of example, sugars, including glucose syrup, modified starches, gelatins, pectins, water, vegetable oils may be used for the preparation of gummy candies, and, for example, flavorings, colorants, and conventional coating agents may also be added.

Alternatively, the compositions of the invention may be in the form of tablets, hard capsules, soft capsules, granules, fine granules, powders, tablets, syrups, emulsions, suspensions and solutions suitable for oral administration. Other suitable pharmaceutical forms may also be used.

Such compositions can be taken alone, for example, when in the form of gummy candies, chewable tablets or chewing gum, or with water when in capsule form, or, especially when in the form of powders or granules, they can be mixed with other foods, for example, with yogurt, creams, gels and the like.

Alternatively, the compositions of the invention can be in ready-to-use beverage form, such as in the form of drinking sachets, of the "stick pack" type, in this case preferably in cream or gel form.

In addition to those already mentioned, the types of pharmaceutical and food additives used in preparing the compositions of the invention, the content ratios of additives to active ingredients, and the methods of preparing the pharmaceutical composition or food supplement may be appropriately selected by the person skilled in the art.

For conventional vehicles and excipients, organic or inorganic substances, or solid or liquid substances may be used as excipients and vehicles, provided they are edible, physiologically acceptable, and compatible with all other components of the composition.

As mentioned, the person skilled in the art is perfectly capable of selecting the most suitable vehicles and excipients for the preparation of the composition.

As examples, organic or inorganic substances, or solid or liquid substances may be used as excipients and vehicles, provided they are edible and pharmaceutically acceptable. Examples of excipients used in the preparation of solid pharmaceutical compositions include, for example, lactose, sucrose, starch, talc, cellulose, dextrins, kaolin, calcium carbonate, stearic acid or magnesium stearate, lactose, polyethylene glycol, mannitol, sorbitol, chelating agents, anti-caking agents, sweetening agents, preservative agents, and flavoring agents.

For the preparation of liquid compositions for oral administration, a conventional inert diluent such as water or an oil, such as a vegetable oil, may be used. The liquid composition may contain, in addition to the inert diluent, auxiliaries such as wetting agents, suspending agents, sweeteners, flavorings, colorants, and preservatives. The liquid composition may be included in capsules of an absorbable material, such as gelatin.

Sweeteners may be one or more natural sugars, optionally reduced, such as sucrose, dextrose, xylitol, mannitol, or sorbitol, or a synthetic product such as sodium saccharin, aspartame, acesulfame k, or sucralose. Acidifying agents may also be added.

Flavoring agents are pharmaceutically acceptable flavors and tastes of synthetic oils or natural oils, the latter extracted from plants, flowers, fruits, and combinations thereof, such as cinnamon, mint, anise, and citrus leaves, bitter almonds, citrus fruits, especially orange and/or lemon, tilia, vanilla, chocolate, and grapefruit oils. Chocolate, vanilla or eucalyptus flavorings and fruit essences, especially apple, pear, peach, strawberry, apricot, orange, lemon and grape, can also be used advantageously.

Examples of the composition of the invention are provided in the Experimental Section below, for illustrative purposes only.

Compositions of the invention may contain from 0.5 mg to 10 mg of melatonin, preferably from 1 mg to 3 mg of melatonin, e.g., 1 mg or 2 mg.

As will be shown in the Experimental Section, the compositions of the invention comprising the granulate of the invention, particularly the compositions in the form of gummy candies, showed a dissolution profile particularly suitable for the treatment of sleep disorders, for the reduction of the time required to fall asleep and for the maintenance of the state of falling asleep.

The expression "sleep disorders" is intended here to include any sleep-related pathology, such as difficulty falling asleep, duration, consolidation, or quality of sleep, and preferably the expression identifies primary insomnia.

The granulate or compositions of the invention are nontoxic and are intended for administration to mammals, preferably humans, in any age group: from adolescents to the elderly.

The granulate or compositions of the invention should be administered shortly before bedtime, e.g., from 15 to 60 minutes earlier, preferably from 30 to 40 minutes earlier. Usually a dose from 1 mg to 3 mg of melatonin, e.g., 1 mg or 2 mg is sufficient to achieve the desired effect. However, these dosages may vary depending on the age, sex, and health status of the person being treated as well as the severity of the disorder.

According to another of its aspects, it is an object of the invention the use of the granulate and composition of the invention in the treatment of sleep disorders as defined above.

According to another of its aspects, it is an object of the invention a method for the treatment of sleep disorders as defined above, which comprises administering an effective amount of the granulate or composition of the invention to a subject in need thereof.

According to another of its aspects, it is an object of the invention a combination consisting of melatonin, sodium alginate and calcium lactate.

According to another of its aspects, it is an object of the invention a combination consisting of melatonin, sodium alginate and calcium lactate for use in a method for the treatment of sleep disorders, as defined above.

According to another of its aspects, it is an object of the invention a combination consisting of melatonin, sodium alginate and calcium lactate as the active ingredient of preferably sustained-release pharmaceutical compositions for use in a method for the treatment of sleep disorders, as defined above.

According to another of its aspects, it is an object of the invention a combination consisting of melatonin, sodium alginate and calcium lactate, as the active ingredient of extended-release granulates, for use in a method for the treatment of sleep disorders, as defined above.

### Experimental Section

### Example 1

### Granulation process

### Step (a)

**Table 1 - Granulation in Diosna mod. P1/6 lab**

| Ingredient | % by weight |
|---|---|
| Melatonin 99% titer | 2.0% |
| Sodium alginate | 49% |
| Calcium lactate | 49% |

Load in order sodium alginate, e.g., about 49% by weight, melatonin, e.g., about 2% by weight, and calcium lactate pentahydrate, e.g., about 49% by weight. Set impeller to 200 rpm and pour in flush purified water qs. After addition is finished, set chopper to 1000 rpm and impeller to 200 rpm for 5 min. Pass wet granulate through 3 mm mesh.

### Drying phase

Dry the granulate in an oven at 60°C for 8 hours. LOD control = 6.3 %

### Calibration phase

Calibrate through 1.2 mm mesh

### Step (b)

**Table 2**

| Ingredient | % by weight |
|---|---|
| Granules (a) from step (a) | 98.12 % |
| Sodium alginate | 0.44% |
| Calcium lactate | 1.44% |

### Step (b')

Granulation in Glatt Fluid Bed mod.Uniglatt

### Coating solution preparation

Solubilize calcium lactate in purified water qs.

### Coating phase

Load the granule obtained from step 1 into the fluidized bed basket. Spray the coating solution by setting the following parameters:
Inlet air Temp.= 40°C - 45°C
Product Temp. = 27°C - 30°C
Spray pressure = 1.5 bar
Liquid flow rate = 15 - 20 g/ min
Air flow = Flap pos. 15

### After finishing the coating solution, dry by setting the following parameters:

Inlet air Temp. = 60°C - 65°C
Product Temp.= 50°C - 55°C
Air flow = Flap pos. 15
LOD control = from 6.5 to 7.5 %.

### Step (b")

Granulation in Glatt Fluid Bed mod.Uniglatt

### Coating solution preparation

Solubilize Sodium Alginate in Water

### Coating phase

Load the granules obtained from step (b') into the fluidized bed basket. Spray the coating solution by setting following parameters:
Inlet air Temp.= 40°C - 45°C
Product Temp. = 27°C - 30°C
Spray pressure = 1.5 bar
Liquid flow rate = 15 - 20 g/ min
Air flow = Flap pos. 15

### After finishing the coating solution drying by setting following parameters:

Inlet air Temp. = 60°C - 65°C
Product Temp. = 50°C - 55°C
Air flow = Flap pos. 15
LOD control = from 6.5 to 7.5 %.

### Calibration phase

Calibrate the granulate through 1.2 mm mesh.

### Example 2

### Granulation process

### Step (a)

**Table 3 - Granulation in Glatt Fluid Bed mod.Uniglatt**

| Ingredient | % by weight |
|---|---|
| Melatonin 99% titer | 10.0 % |
| Sodium alginate | 45% |
| Calcium lactate | 45% |

In this case, a single granulation step is made in Glatt Fluid Bed with all ingredients and 40% purified water.

### Coating phase

Load melatonin, e.g., about 10% by weight, sodium alginate, e.g., 45% by weight, and calcium lactate, e.g., 45% by weight, into the fluid bed basket. Spray the purified water by setting the following parameters:
Inlet air Temp. = 40°C - 45°C
Product Temp. = 27°C - 30°C
Spray pressure = 1.5 bar
Liquid flow rate = 15 - 20 g/ min
Air flow = Flap pos. 15

### After finishing the wetting phase with purified water, dry by setting following parameters:

Inlet air Temp.= 60°C - 65°C
Product Temp. = 50°C - 55°C
Air flow = Flap pos. 15
LOD control = from 6.5 to 7.5 %.

### Calibration phase

Calibrate the granulate through 1.2 mm mesh
The resulting granulate has the following specifications:
   GRANULOMETRY:
   No more than 10%: ≥ 710 µm
   80% between 125 µm and 710 µm
   Not more than 10 %: < 125 µm
   LOD (70°C): 6.0 % - 8.0 %.
   Density: 0.4 to 0.6 g/ml
   Flowability (100 grams): ≤ 25 sec.

### Example 3

A composition of the invention is prepared in the form of a gummy candy
MOULD-FORM "menthol boluses"
WEIGHT SINGLE CANDY Single candy: about 2 g.

### Ingredients:

Granulate obtained from Example 1, glucose syrup, sugar, modified starches, water, vegetable oil (coconut, canola), flavoring, coloring agent: E133, coating agent: carnauba wax.

### Example 4

### Dissolution tests

### Example 4.1

Dissolution test according to USP (500 mL 0.1 N HCl, 37°C, 100 rpm)

**Table 4**

| Time | Candies from Example 3 divided into 2 parts | Candies from Example 3 divided into small pieces |
|---|---|---|
| 30 min | 19.3% | 29.6% |
| 2 hours | 40.0% | 71.6% |
| 4 hours | 54.7% | 76.2% |

### Example 4.2

Dissolution test (900 mL buffer pH 7, 37°C, 100 rpm)

**Table 5**

| Time | Candies from Example 3 divided into 2 parts | Candies from Example 3 divided into small pieces |
|---|---|---|
| 30 min | 29.8% | 41.3% |
| 2 hours | 57.0% | 55.6% |
| 4 hours | 63.2% | 71.1% |

### EXPERIMENTAL PART

Alginates are naturally occurring linear copolymers extracted from the cell wall of the brown algae *Laminaria* and *Ascophillum.* Alginates from algae must undergo a series of treatments before they can actually be used for applications where their high purification is required. Since the alginate salts found in algae are insoluble, their transformation into sodium alginate, which is soluble instead, is necessary.

Alginates encounter considerable application interest primarily because of their ability to form, in the presence of bivalent cations (e.g., Ca²⁺) polymeric gels. The application interest in alginates originates mainly from their ability to form gels, and the study of the gelation process assumes paramount importance.

A gel is a solid consisting of a three-dimensional network of molecules held together by junction zones (either physical or chemical in nature) to form a solid phase in which the dispersed liquid medium remains embedded. The network gives the gel the characteristic properties of a solid, while the liquid phase controls its density. These polymers, placed in solution in the presence of bivalent cations, induce the establishment of ionic bonds between the different polymer chains, promoting a sol-gel transition.

Ca²⁺ cations bind, first, the G groups until the available binding sites are saturated. Next, they proceed to bind with M groups, which consist of monomeric units of mannuronic acid (at least at the macroscopic level and, especially, when neighboring units consist of G residues) until the association of the chains is limited by the network. The explanation for this observation lies in the different ability to form junction zones with the Ca²⁺ cations of M and G blocks. The latter are both polyanionic and will form intermolecular anionic bonds with bivalent cations. However, G blocks are also able to chelate Ca²⁺ because of their spatial organization, forming a binding site between two adjacent G blocks and thus giving rise to a stronger interaction. Thus, dimeric junctions rather than aggregates are formed. Hence the "egg-box" model, which takes its name from the characteristic shape of the molecular structure that is created

A relative alginate affinity scale was, in addition, established for several bivalent cations of interest:

Pb²⁺>Cu²⁺>Cd²⁺> Ba²⁺>Sr²⁺>Ca²⁺>Co²⁺=Ni²⁺=Zn²⁺>Mn²⁺>Mg²⁺

Among all possible bivalent cations, Ca²⁺ was selected, both according to affinity and taking safety into account. When using alginate gels, one of the main problems is low chemical stability.

Substances with a high affinity for them (such as phosphates or citrates) may sequester calcium ions and destabilize the initial gel. The gel may also be destabilized by the presence of other non-gelling cations.

For these reasons, the choice of calcium lactate does not seem obvious but is rather the result of careful selection reasoning that takes into account the chemical and physical properties of calcium lactate itself and the other components present in the granulate composition (sodium alginate and melatonin).

Of all the possible calcium salts, calcium lactate was selected because of a number of characteristics that make calcium lactate the salt of choice for the preparation of melatonin granulate:
- good solubility in water
- pH of 5% calcium lactate solution in water: 6-8
- Ca titer: from 13.4% to 14.5%

The other possible calcium salts have been excluded either because they are insoluble in water (e.g., calcium carbonate) or because they are poorly soluble (e.g., calcium citrate) or because, although soluble, once dissolved in water they result in a pH value that is incompatible with the other two ingredients in the granulate composition (melatonin and sodium alginate).

The choice could also have fallen on a salt formed by the binding of lactic acid with a bivalent cation that is equally safe as Ca²⁺, namely Mg²⁺. The salt formed when lactic acid binds the bivalent cation Mg²⁺ is magnesium lactate. Magnesium lactate turns out to be very similar to calcium lactate in terms of its chemical and physical characteristics:
- good solubility in water;
- pH of 5% magnesium lactate solution in water: 6.5-8.5;
- Mg titer: from 10.0% to 10.5%.

However, magnesium lactate, despite having similar chemical and physical characteristics to calcium lactate, is not considered to be a viable candidate, equivalent to calcium lactate in terms of functionality within the retard granulate, which includes the presence of melatonin and sodium alginate, because the Mg²⁺ ion does not possess gelling properties, as the Ca²⁺ ion has.

To provide experimental evidence, a granulate was produced wherein calcium lactate was substituted 1:1 for magnesium lactate. The composition tested, the granulation process applied and the outcome of the test are given below:

| Ingredient | % |
|---|---|
| Melatonin | 10.00 |
| Sodium alginate | 45.00 |
| Magnesium lactate | 45.00 |

A single granulation step is made in Glatt Fluid Bed with all ingredients and 40% purified water.

### Coating phase

Load into the fluid bed basket melatonin, e.g. about 10% by weight, sodium alginate, e.g. 45% by weight, magnesium lactate, e.g. 45% by weight.

Spray purified water by setting the following parameters:
Inlet air temperature = 40°C - 45°C
Product temperature = 27°C - 30°C
Spray pressure = 1.5 bar
Liquid flow rate = 15 - 20 g/min
Air flow = Flap pos. 15

After finishing the wetting phase with purified water, dry by setting the following parameters:
Inlet air temperature = 60°C - 65°C
Product temperature = 50°C - 55°C
Air flow = Flap pos. 15
LOD control = 6.5 to 7.5 %.
Calibration phase
Calibrate granulate through 1.2 mm mesh

The resulting granulate has the following specifications:

### GRANULOMETRY:

No more than 10%: ≥ 710 µm
80% between 125 µm and 710 µm
No more than 10 %: < 125 µm
LOD (70°C): 6.0 % - 8.0 %.
Density: 0.45 to 0.65 g/ml
Flowability (100 grams): ≤ 25 sec.

The granulate obtained using magnesium lactate has chemical and physical characteristics superimposed on the granulate obtained using calcium lactate.

To test the gelling property of the granulate containing magnesium lactate, which is responsible for the functionality of the melatonin granulate, 1 gram of granulate with calcium lactate and 1 gram of granulate with magnesium lactate were poured into 150 ml of HCl 0.1N to simulate the gastric environment.

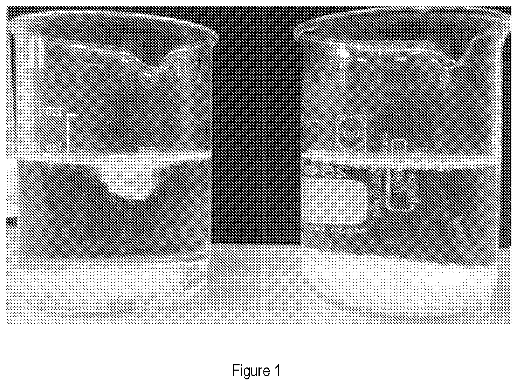

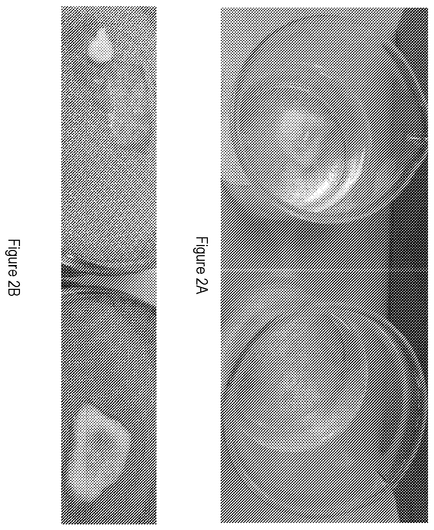

Figure 1 and Figures 2A and 2B show photos of the test outcome:
Figure 1 (on the left granulate with magnesium lactate and on the right granulate with calcium lactate) shows the behavior of the 2 granulate just poured into HCl 0.1N.
Figure 2A and 2B shows the behavior of the 2 granulate just poured into HCl 0.1N (top view). On the left granulate with magnesium lactate and on the right granulate with calcium lactate.

As can be seen from the photos, the melatonin granulate with calcium lactate, in contact with the HCl 0.1N solution, hydrates evenly and the powder granules gel smoothly and evenly, without forming aggregates. Granulate with magnesium lactate, on the other hand, hydrates with difficulty and longer times, and tends to form large lumpy aggregates, and, the part that hydrates, forms a gel with irregular characteristics and therefore unsuitable for the formation of a gel layer around the granular powder granule.

## Claims

1. A slow-release granulate that comprises or, alternatively, consists of melatonin, sodium alginate, and calcium lactate.

2. The granulate according to claim 1, **characterized by** the fact that said granulate comprises from 1% to 20% by weight of melatonin with respect to the total weight of the granulate, preferably from 1.5% to 15%, more preferably from 2% to 10%; from 20% to 60% by weight of sodium alginate with respect to the total weight of the granulate, preferably from 40% to 55%, more preferably from 45% to 50%; and from 30 to 60% by weight of calcium lactate with respect to the total weight of the granulate, preferably from 40% to 55%, more preferably from 45% to 50%.

3. A process for preparing a granulate according to claim 1 or 2, wherein said process comprises the following steps:
a. a wet granulation of a mixture comprising or, alternatively, consisting of melatonin, sodium alginate and calcium lactate to form granules intended to be coated;
b. a coating of the granules obtained from step (a) through two sequential steps:
b' a granulation of the granules obtained from passage (a) with calcium lactate; and
b" a granulation of the granules obtained from passage (b') with sodium alginate.

4. A process for preparing a granulate according to claim 1 or 2, wherein said process comprises a granulation of a mixture comprising or, alternatively, consisting of melatonin, sodium alginate and calcium lactate and subsequent coating with a mixture comprising or, alternatively, consisting of sodium alginate and calcium lactate.

5. A combination consisting of melatonin, sodium alginate and calcium lactate.

6. A pharmaceutical and/or food composition comprising the granulate according to claim 1 or 2, or the combination of claim 5, together with one or more conventional excipients and/or vehicles.

7. The composition according to claim 6, **characterized by** the fact that it is in the form of a composition for oral use.

8. The composition according to claim 7, **characterized by** the fact that it is in the form of gummy candies, chewable tablets, capsules or chewing gum.

9. The composition according to any one of claims 6 to 8, **characterized by** the fact that it comprises from 0.5 to 5 mg of melatonin, preferably from 1 to 3 mg of melatonin.

10. The granulate according to claim 1 or 2, the combination of claim 5, or the composition according to any one of claims 6 to 9, for use in a method of treatment of sleep disorders, preferably primary insomnia, for the reduction of the time required to fall asleep and for the maintenance of the state of falling asleep.

## Patentansprüche

1. Granulat mit verzögerter Freisetzung, das Melatonin, Natriumalginat und Calciumlactat umfasst oder alternativ daraus besteht.

2. Granulat nach Anspruch 1, **dadurch gekennzeichnet, dass** das Granulat von 1 bis 20 Gew.- % Melatonin, bezogen auf das Gesamtgewicht des Granulats, bevorzugt von 1,5 bis 15 %, bevorzugter von 2 bis 10 %, von 20 bis 60 Gew.- % Natriumalginat, bezogen auf das Gesamtgewicht des Granulats, bevorzugt von 40 bis 55 %, bevorzugter von 45 bis 50 %, und von 30 bis 60 Gew.- % Calciumlactat, bezogen auf das Gesamtgewicht des Granulats, bevorzugt von 40 bis 55 %, bevorzugter von 45 bis 50 %, umfasst.

3. Verfahren zur Herstellung eines Granulats nach Anspruch 1 oder 2, wobei das Verfahren die folgenden Schritte umfasst:
a. eine Nassgranulierung einer Mischung, die Melatonin, Natriumalginat und Calciumlactat umfasst oder alternativ daraus besteht, um Granulate zu bilden, die beschichtet werden sollen;
b. eine Beschichtung der aus Schritt (a) erhaltenen Granulate durch zwei aufeinanderfolgende Schritte:
b' eine Granulierung der aus Schritt (a) erhaltenen Granulate mit Calciumlactat; und
b" eine Granulierung der aus Schritt (b') erhaltenen Granulate mit Natriumalginat.

4. Verfahren zur Herstellung eines Granulats nach Anspruch 1 oder 2, wobei das Verfahren eine Granulierung einer Mischung, die Melatonin, Natriumalginat und Calciumlactat umfasst oder alternativ daraus besteht, und eine anschließende Beschichtung mit einer Mischung, die Natriumalginat und Calciumlactat umfasst oder alternativ daraus besteht, umfasst.

5. Kombination, bestehend aus Melatonin, Natriumalginat und Calciumlactat.

6. Pharmazeutische und/oder Nahrungsmittelzusammensetzung umfassend das Granulat nach Anspruch 1 oder 2 oder Kombination nach Anspruch 5 zusammen mit einem oder mehreren herkömmlichen Hilfsstoffen und/oder Vehikeln.

7. Zusammensetzung nach Anspruch 6, **dadurch gekennzeichnet, dass** sie in Form einer Zusammensetzung zur oralen Anwendung vorliegt.

8. Zusammensetzung nach Anspruch 7, **dadurch gekennzeichnet, dass** sie in Form von Gummibonbons, Kautabletten, Kapseln oder Kaugummi vorliegt.

9. Zusammensetzung nach einem der Ansprüche 6 bis 8, **dadurch gekennzeichnet, dass** sie von 0,5 bis 5 mg Melatonin, bevorzugt von 1 bis 3 mg Melatonin enthält.

10. Granulat nach Anspruch 1 oder 2, Kombination nach Anspruch 5 oder Zusammensetzung nach einem der Ansprüche 6 bis 9 zur Verwendung in einem Verfahren zur Behandlung von Schlafstörungen, bevorzugt primärer Schlaflosigkeit, zur Verkürzung der zum Einschlafen erforderlichen Zeit und zur Aufrechterhaltung des Einschlafzustands.

## Revendications

1. Granule à libération lente qui comprend ou, en variante, est constituée de mélatonine, d'alginate de sodium et de lactate de calcium.

2. Granule selon la revendication 1, **caractérisée en ce que** ladite granule comprend de 1 à 20 % en poids de mélatonine par rapport au poids total de la granule, de préférence de 1,5 à 15 %, plus préférablement de 2 à 10 % ; de 20 à 60 % en poids d'alginate de sodium par rapport au poids total de la granule, de préférence de 40 à 55 %, plus préférablement de 45 à 50 % ; et de 30 à 60 % en poids de lactate de calcium par rapport au poids total de la granule, de préférence de 40 à 55 %, plus préférablement de 45 à 50 %.

3. Procédé de préparation d'une granule selon la revendication 1 ou 2, dans lequel ledit procédé comprend les étapes suivantes :
a. une granulation par voie humide d'un mélange comprenant ou, en variante, constitué de mélatonine, d'alginate de sodium et de lactate de calcium pour former des granules destinées à être enrobées ;
b. un enrobage des granules obtenues à l'étape (a) par l'intermédiaire de deux étapes séquentielles :
b' une granulation des granules obtenues au passage (a) avec du lactate de calcium ; et
b" une granulation des granules obtenues au passage (b') avec de l'alginate de sodium.

4. Procédé de préparation d'une granule selon la revendication 1 ou 2, dans lequel ledit procédé comprend une granulation d'un mélange comprenant ou, en variante, consistant en de la mélatonine, de l'alginate de sodium et du lactate de calcium, suivie d'un enrobage avec un mélange comprenant ou, en variante, consistant en de l'alginate de sodium et du lactate de calcium.

5. Combinaison constituée de mélatonine, d'alginate de sodium et de lactate de calcium.

6. Composition pharmaceutique et/ou alimentaire comprenant la granule selon la revendication 1 ou 2, ou combinaison selon la revendication 5, conjointement avec un ou plusieurs excipients et/ou véhicules classiques.

7. Composition selon la revendication 6, **caractérisée par le fait qu'**elle se présente sous la forme d'une composition à usage oral.

8. Composition selon la revendication 7, **caractérisée par le fait qu'**elle se présente sous la forme de bonbons gélifiés, de comprimés à croquer, de gélules ou de chewing-gums.

9. Composition selon l'une quelconque des revendications 6 à 8, **caractérisée par le fait qu'**elle comprend de 0,5 à 5 mg de mélatonine, de préférence de 1 à 3 mg de mélatonine.

10. Granule selon la revendication 1 ou 2, combinaison selon la revendication 5, ou composition selon l'une quelconque des revendications 6 à 9, destinée à être utilisée dans un procédé de traitement des troubles du sommeil, de préférence de l'insomnie primaire, pour la réduction du temps nécessaire à l'endormissement et pour maintenir l'état d'endormissement.
